# EUROPEAN PATENT SPECIFICATION

(11) **EP 2 037 756 B1**
(45) Date of publication and mention of the grant of the patent: **02.09.2015**
(21) Application number: 07794830.5
(22) Date of filing: 14.05.2007
(51) Int. Cl.: A23K 1/14, A23K 1/16, A23K 1/175, A23K 1/18

(54) **COMPOSITIONS AND METHODS FOR PREVENTING OR TREATING CHRONIC RENAL FAILURE IN FELINES WITH HYPERTHYROIDISM**
ZUSAMMENSETZUNGEN UND VERFAHREN ZUR VORBEUGUNG ODER BEHANDLUNG VON CHRONISCHEM NIERENVERSAGEN BEI KATZEN MIT SCHILDRÜSENÜBERFUNKTION
COMPOSITIONS ET PROCÉDÉS SERVANT À PRÉVENIR OU À TRAITER L'INSUFFISANCE RÉNALE CHRONIQUE CHEZ DES FÉLINS SOUFFRANT D'HYPERTHYROÏDIE

(30) Priority: 12.05.2006 US 800066 P
(43) Date of publication of application: 25.03.2009
(73) Proprietor: Hill's Pet Nutrition, Inc., Topeka, KS 66603 (US)
(72) Inventor: WEDEKIND, Karen, Joy, Meriden, KS 66512 (US); ALLEN, Timothy, Arthur, Lawrence, KS 66049 (US); FRITSCH, Dale, Allen, Topeka, KS 66610 (US); DODD, Chadwick, Everett, Lawrence, KS 66049 (US)
(74) Representative: Daniels, Jeffrey Nicholas
(86) International application number: PCT/US2007/011512
(87) International publication number: WO 2007/133726

(56) References cited:
- WO-A-2004/112498
- WO-A-2004/112499
- WO-A-2007/084986
- WO-A-2007/087623
- KONTESSIS P ET AL: "RENAL METABOLIC AND HORMONAL RESPONSES TO INGESTION OF ANIMAL AND VEGETABLE PROTEINS" KIDNEY INTERNATIONAL, vol. 38, no. 1, 1990, pages 136-144, XP009090725 ISSN: 0085-2538

## Description

### BACKGROUND OF THE INVENTION

### Field of the Invention

The invention relates generally to compositions for use in preventing or treating chronic renal failure (CRF) and particularly to compositions for use in preventing or treating CRF in felines with hyperthyroidism.

### Description of the Prior Art

Both hyperthyroidism and CRF (also known as chronic renal disease or renal insufficiency) are common diseases, particularly in older cats. Hyperthyroidism is characterized by hypermetabolism of the thyroid gland and excessive production of the thyroid hormones triiodothyronine (T3) and tetraiodothyronine (T₄). Hyperthyroidism can mask concurrent renal disease by, for example, increasing glomerular flow rate (GRF). In addition, some anti-thyroid medications can cause kidney damage thereby resulting in the development of a kidney disease. CRF is a progressive, terminal kidney disease that is one of the leading causes of death in felines.

Thus, there is a need for compositions and methods for treating CRF in felines with hyperthyroidism that provide partial or complete relief. In addition, there is a need for compositions and methods for preventing CRF in felines with hyperthyroidism.

WO2004/112948 discloses methods for treating hyperthyroidism in a cat and compositions comprising limited amounts of iodine.

WO2004/112499 discloses methods for preventing hyperthyroidism in a cat and compositions comprising limited amounts of selenium and iodine.

Kontessis et al. Kidney International, Vol. 38 (1990) pages 136-144 discloses renal, metabolic and hormal responses to ingestion of animal and vegetable proteins.

WO2007/084986 discloses compositions and methods for preventing or treating feline chronic kidney disease.

WO2007/087623 discloses methods and compositions for treating feline hyperthyroidism.

### SUMMARY OF THE INVENTION

It is an object of the invention to provide compositions for use in preventing CRF in felines with hyperthyroidism.

Specifically, the present invention provides a composition for use in preventing or treating chronic renal failure in a feline with hyperthyroidism,
wherein if the composition is for use in preventing chronic renal failure, the composition comprises from 28 to 35% protein on a dry matter basis wherein the protein comprises at least 75% vegetable protein; and preferably, wherein the composition comprises from 31 to 35% protein,
wherein if the composition is for use in treating chronic renal failure, the composition comprises from 28 to 30% protein on a dry matter basis, wherein the protein comprises at least 75% vegetable protein,
and wherein the composition for use in preventing or treating chronic renal failure further comprises from 0.1 to less than 1 mg/kg iodine on a dry matter basis and/or from 0.1 to 1 mg/kg selenium on a dry matter basis.

The present invention further provides use of a combination of vegetable protein, selenium and iodine in the manufacture of a food composition to treat and/or prevent chronic renal failure in a feline having hyperthyroidism, wherein the amount of protein in the composition is from 25% to 35% protein on a dry matter basis, which comprises at least 75% vegetable protein, wherein the amount of selenium in the composition is 0.1 to 1 mg/kg selenium on a dry matter basis, and
wherein the amount of iodine in the composition is 0.1 to 1 mg/kg iodine on a dry matter basis.

It is another object of the invention to provide compositions suitable for use in treating CRF in felines with hyperthyroidism.

The description provides methods for preventing CRF in felines with hyperthyroidism and methods for treating CRF in felines with hyperthyroidism.

The description further provides articles of manufacture comprising a composition as defined above or two or more ingredients that, when combined together and, optionally, with additional ingredients, yield a composition as defined above for use in preventing or treating CRF in a feline with hyperthyroidism.

The description additionally provides means for communicating information about the compositions, methods, and articles of manufacture of the invention.

Additional objects, features, and advantages of the invention will be apparent to those skilled in the art

### DETAILED DESCRIPTION QF THE INVENTION

CRF is a progressive -kidney disease that has four phases: loss of renal reserve, renal insufficiency, azotemia, and uremia. The kidneys have a large built-in reserve as only ∼30% of kidney capacity is needed for normal kidney function. Kidney capacity diminishes with time for a variety of reasons, for example, advanced age and diseases and medications that damage the kidney(s). Renal insufficiency is characterized by decrease in renal function, and is generally observed when about 70% of kidney function has been lost (*i.e.,* when only about 30% of kidney capacity is available). Clinical signs are typically not obvious during the phases of loss of renal reserve and renal insufficiency, thereby making it difficult to detect CRF. Azotemia, the third phase of CRF, is characterized by an abnormally high concentration of urea, creatinine, and/or other non-protein nitrogen-containing substances in the blood. Renal function is markedly reduced in the azotemic phase, although clinical signs may be mild and can go undetected. Uremia is characterized by the presence of abnormal quantities of urine or urine constituents in blood. The uremic phase is characterized by overt clinical signs.

Hyperthyroidism is characterized by hypermetabolism of the thyroid gland and the excessive production of the thyroid hormones T₃ and T₄. Most of T₃ and T₄ are bound to serum proteins. The portion of T₃ and T₄ partitioned into serum, and not associated with protein, is called free T₃ (fT₃) and T₄ (fT₄). One skilled in the art can accurately diagnose hyperthyroidism in a feline utilizing thyroid function tests, examining clinical signs, and/or observing the animal's response to trial thyroid hormone administration. Thyroid function tests are known to those skilled in the art and include, for example, tests for determining the concentrations of total and free serum T₃ and T₄, tests for determining the concentration of thyroid stimulating hormone, and the sodium pertechnetate and T₃ suppression tests. See, for example, Small Animal Nutrition, pages 863-868 (2000).

CRF is a terminal disease in felines. It is, therefore, important to prevent decrease in renal function in felines that have been diagnosed with hyperthyroidism, but do not show clinical signs of CRF. Thus, in one aspect, the present invention provides a composition for use in preventing CRF in a feline with hyperthyroidism that may be susceptible to develop renal insufficiency. Such a feline is one that has not been diagnosed with CRF, but is at risk to develop CRF due to, for example, advanced age, genetic predisposition, or disease(s) or medication(s) that can damage the feline's kidney(s). Preventing CRF includes reducing the risk of, delaying the onset of, and/or keeping a hyperthyroid feline from developing CRF. Thus, feeding a composition for preventing CRF can reduce a feline's risk of developing CRF. In addition, feeding such a composition can slow down the progression of loss of a feline's kidneys' built-in reserve, thereby delaying the onset of the later phases of CRF.

The composition for use in preventing CKF is suitable for any feline with hyperthyroidism that is susceptible to develop CKF. In some embodiments, the feline is a companion feline. A companion feline can be a feline kept as a pet. A companion feline can also be a feline from a widely domesticated species, for example, cats (*Felis domesticus*) regardless of whether or not it is kept as a pet. In some embodiments, the feline is an adult feline. An adult feline is a feline of any age after juvenile growth and development has been completed, including senior and geriatric felines. For example, an adult cat typically is one that is from about one year old through the remainder of its life. A senior feline is one of an age at which it is at a risk for suffering from an age-related disease regardless of whether or not the feline shows obvious physical or Behavioral signs of aging. For example, a senior cat typically is a cat from about seven to about eleven years old. A geriatric feline is a feline showing-signs of aging. For example, a geriatric cat typically is a cat of about twelve years of age and beyond.

This composition for use in preventing CKF in a feline with hyperthyroidism comprises from 28 to 35% protein. The protein present in the composition comprises at least 75% vegetable protein. The composition also comprises from 0.1 to less than 1 mg/kg iodine and/or from 0.1 to 1 mg/kg selenium on a dry matter basis.

Commercial adult cat foods, for example, typically comprise from about 35 to about 45% protein. Thus, in many embodiments, a composition for use in preventing CRF in a hyperthyroid feline comprises less protein than most commercially available adult cat foods.

In some embodiments, the composition for use in preventing CRF in a hyperthyroid feline comprises from about 28 to about 31% protein. In some such embodiments, the composition comprises from about 28 to about 30% protein; and in other such embodiments, the composition comprises from about 29 to about 31% protein. In yet other such embodiments, the composition comprises from about 28 or about 28.5 to about 29, about 29.5, about 30, about 30.5, or about 31% protein.

In other embodiments, the composition for use in preventing CKF in a hyperthyroid feline comprises from about 31 to about 35% protein. In some such embodiments, the composition comprises from about 32 to about 34% protein. In other embodiments, the composition comprises from about 32 to about 35% protein. In other embodiments, the composition comprises from about 33 to about 35% protein, hi other embodiments, the composition comprises from about 31, about 32, or about 33 to about 34 or about 35% protein. In further embodiments, the composition comprises from about 31 to about 32, about 33, about 34, or about 35% protein.

Dietary protein restriction is beneficial for preventing and treating CRF. Excessive protein is catabolized to urea and other nitrogenous compounds normally excreted by the kidneys. Decreased renal function leads to accumulation of these compounds. Endogenous proteins will be degraded if amino acid intake is insufficient to maintain nitrogen balance. Thus, a composition for use in preventing and/or treating CRF in a feline with hyperthyroidism can facilitate achieving nitrogen balance, and can help limit the accumulation of waste products by decreasing protein intake.

Because felines are carnivores, feline foods typically are formulated to comprise mostly animal and fish protein. To the contrary, the protein in a composition according to the invention for use in preventing CRF in a feline with hyperthyroidism according to the invention comprises at least about 75% vegetable protein. In some such embodiments, the composition comprises at least about 80% vegetable protein. In other embodiments, the composition comprises at least about 85% vegetable protein. In other such embodiments, the protein comprises at least about 90% vegetable protein. And in other such embodiments, the protein comprises at least about 95% vegetable protein *(i.e.,* from at least about 95 up to about 100% vegetable protein).

Thus, in some embodiments, the composition for use in preventing CRF in a feline with hyperthyroidism comprises from about 28 to about 31% protein wherein the protein comprises at least about 75% vegetable protein. In some such embodiments, the composition comprises from about 28 to about 30% protein wherein the protein comprises at least about 75, at least about 80, at least about 85, at least about 90, or at least about 95% vegetable protein. In other such embodiments, the composition comprises from about 29 to about 31% protein wherein "the protein comprises at least about 75, at least about 80, at least about 85, at least about 90, or at least about 95% vegetable protein. In yet other such embodiments, the composition comprises from about 28 or about 28.5 to about 29, about 29.5, about 30, about 30.5, or about 31% protein wherein the protein comprises at least about 75, at least about 80, at least about 85, at least about 90, or at least about 95% vegetable protein.

In other embodiments, the composition for use in preventing CRF in a feline with hyperthyroidism comprises from about 31 to about 35% protein wherein the protein comprises at least about 75% vegetable protein. In some such embodiments, the composition comprises from about 31, about 32, or about 33 to about 34 or about 35% protein wherein the protein comprises at least about 75, at least about 80, at least about 85, at least about 90, or at least about 95% vegetable protein. In other embodiments, the composition comprises from about 31 to about 32, about 33, about 34, or about 35% protein, and the protein comprises at least about 75, at least about 80, at least about 85, at least about 90, or at least about 95% vegetable protein. In yet other embodiments, the composition comprises from about 28 or about 28.5 to about 29, about 29.5, or about 30% protein, and the protein comprises at least about 75, at least about 80, at least about 85, at least about 90, or at least about 95% vegetable protein.

In some embodiments, the composition for use in preventing CRF in a feline with hyperthyroidism comprises from about 0.1 to less than about 1 mg/kg iodine, hi some such embodiments, the composition comprises from about 0.1 to about 0.5 mg/kg iodine. In other such embodiments, the composition comprises from about 0.1 to about 0.3 mg/kg iodine. In yet other such embodiments, the composition comprises from about 0.15 to about 0.25 mg/kg iodine. And in further such embodiments, the composition comprises from, about 0.1 to about 0.2 mg/kg iodine. Iodine is a constituent of T₃ and T₄. The thyroid glands actively trap iodine to ensure an adequate supply of thyroid hormones. Iodine as used herein refers to the iodine atom without reference to its molecular or ionic form, and includes iodine present in one or more chemical forms such as, for example, iodide, iodate, and periodate.

In some embodiments, the composition for use in preventing CRF in a feline with hyperthyroidism comprises from about 0.1 to about 1 mg/kg selenium. In some such embodiments, the composition comprises from about 0.1 to about 0.8 mg/kg selenium. In other such embodiments, the composition comprises from, about 0.15 to about 0.65 mg/kg selenium. In yet other such embodiments, the composition comprises from about 0.4 to about 0.7 mg/kg selenium. In further such embodiments, the composition comprises from about 0.3 to about 0.65 mg/kg selenium. Selenium has a role in maintaining normal thyroid and iodine metabolism, particularly through the control of the deiodinase enzymes that regulate the conversion of T₄ to T₃. Selenium as used herein refers to the selenium atom without reference to its molecular or ionic form, and includes selenium present in one or more chemical forms such as, for example, selenide, selenite, and selenate.

In some embodiments, the composition for use in preventing CRF in a feline with hyperthyroidism comprises from about 0.1 to less than about 1 mg/kg iodine as well as from about 0.1 to less than about 1 mg/kg selenium. In some such embodiments, the composition comprises from about 0.1 to about 0.5 mg/kg iodine and from about 0.1 to about 0.8 mg/kg selenium. In other such embodiments, the composition comprises from about 0.1 to about 0.3 mg/kg iodine and from about 0.15 to about 0.65 mg/kg selenium. In further such embodiments, the composition comprises (1) from about 0.1, about 0.15, or about 0.2 to about 0.25, about 0.3, or about 0.5 mg/kg iodine, and (2) from about 0.1, about 0.15, about 0.3, or about 0.4 to about 0.65, about 0.7, or about 0.8 mg/kg selenium.

Tables 2 and 3 of U.S. Patent Application No. US 2005/0058691 A1 list the iodine and selenium content of commercially available canned and dry cat foods. The average amounts of selenium in the 28 tested canned foods and 14 tested dry foods were 1.77 and 0.69 mg/kg, respectively, with many foods having more than 2 mg/kg. The average amounts of iodine in those foods were 7.83 mg/kg and 2.77 mg/kg, respectively, with some foods having more than 30 mg/kg. Thus, the compositions comprise amounts of iodine and/or selenium that are lower (and in some embodiments, much lower) than the amounts of iodine and selenium in many commercially available foods. Thus, in some embodiments, feeding a composition to a feline results in restricting the feline's intake of iodine. In other embodiments, feeding a composition to a feline results in restricting the feline's intake of selenium. In further embodiments, feeding a composition to a feline results in restricting the feline's intake of both iodine and selenium.

One skilled in the art would understand that a composition for use in preventing CRF in a feline with hyperthyroidism can comprise any of the combinations of protein (with varying amounts of vegetable protein), iodine, and selenium discussed above.

For example, in some embodiments, the composition for use in preventing CRP in a feline with hyperthyroidism comprises from about 29 to about 31% protein wherein the protein comprises at least about 75, at least about 80, at least about 85, at least about 90, or at least about 95% vegetable protein, and (1) from about 0.1, about 0.15, or about 0.2 to about 0.25, about 0.3, or about 0.5 mg/kg iodine, and/or (2) from about 0.1, about 0.15, about 0.3, or about 0.4 to about 0.65, about 0.7, or about 0.8 mg/kg selenium.

In other embodiments, the composition for use in preventing CKF in a feline with hyperthyroidism from about 28 or about 28.5 to about 29, about 29.5, about 30, about 30.5, or about 31% protein wherein the protein, comprises at least about 75, at least about 80, at least about 85, at least about 90, or at least about 95.% vegetable protein, and (1) from about 0.1, about 0.15, or about 0.2 to about 0.25, about 0.3, or about 0.5 mg/kg iodine, and/or (2) from about 0.1, about 0.15, about 0.3, or about 0.4 to about 0.65, about 0.7, or about 0.8 mg/kg selenium.

In other embodiments, the composition for use in preventing CRF in a feline with hyperthyroidism comprises from about 31, about 32, or about 33 to about 34 or about 35% protein, wherein the protein comprises at least about 75, at least about 80, at least about 85, at least about 90, or at least about 95% vegetable protein, and (1) from about 0.1, about 0.15, or about 0.2 to about 0.25, about 0.3, or-.about 0.5 mg/kg iodine, and/or (2) from about 0.1, about 0.15, about 0.3, or about 0:4 to about 0.65, about 0.7, or about 0.8 mg/kg selenium.

In other embodiments, the composition for use in preventing CRF in a feline with hyperthyroidism comprises from about 28 or about 28.5 to about 29, about 29.5, or about 30% protein, and the protein comprises at least about 75, at least about 80, at least about 85, at least about 90, or at least about 95% vegetable protein, and (1) from about 0.1, about 0.15, or about 0.2 to about 0.25, about 0.3, or about 0.5 mg/kg iodine, and/or (2) from about 0.1, about 0.15, about 0.3, or about 0.4 to about 0.65, about 0.7, or about 0.8 mg/kg selenium.

Vegetarian diets generally contain higher amounts of fiber, magnesium, folic acid, vitamin C, vitamin D, and carotenoids than non-vegetarian diets. Vegetarian diets generally contain less saturated fat, cholesterol, protein, sulfur-containing amino acids, calcium, and iodine than non-vegetarian diets. Phosphorus from vegetable protein is less available than phosphorus from animal protein. Thus, some characteristics of vegetarian diets (e.g., lower amount of protein, lower bioavailability of phosphorus, lower amount of sulfur-containing amino acids thereby reducing diet acidity) are consistent with the goals of the invention to prevent and/or treat CRF in felines. Plant ingredients suitable for preparing compositions of the invention for preventing and/or treating CRF include, for example, potato concentrate, soy concentrate, soy protein isolate, soybean meal, corn gluten meal, rice protein isolate, pea protein concentrate, wheat protein concentrate, and wheat protein isolate.

In some embodiments, the composition for use in preventing CRF in a feline with hyperthyroidism further comprises from about 0.45 to about 0.8% phosphorus. In some such embodiments, the composition comprises about 0.45 to about 0.6% phosphorus, for example, from about 0.45 to about 0.55% or from about 0.45 to about 0.5% phosphorus. In other such embodiments, the composition comprises from about 0.6 to about 0.8% phosphorus, for example, from about 0.7 to about 0.8% or from about 0.6 to about 0.75% phosphorus. Phosphorus as used herein refers to the phosphorus atom without reference to its molecular or- ionic farm. Secondary hyperparathyroidism and phosphorus retention have been incriminated as causes of CRF. Therefore, reduction of phosphorus in a feline's diet can be beneficial for preventing and/or treating CRF.

In some embodiments, the composition for use in preventing CRF in a feline with hyperthyroidism further comprises from about 0.2 to about 0.35% sodium. In some such embodiments, the composition comprises from about 0.2 to about 0.25% sodium. In other such embodiments, the composition comprises from about 0.25 to about 0.3% sodium. Sodium as used herein refers to the sodium atom without reference to its molecular or ionic form. When dietary sodium intake changes, fractional- excretion of sodium must change as well to maintain sodium balance. Felines with CRF can only vary sodium excretion over a limited range, which narrows progressively as glomerular filtration rate declines. Thus, such felines cannot tolerate excessively high or excessively low dietary sodium intake. Feeding a feline a composition for preventing-and/or treating CRF can help maintain optimal sodium balance.

In some embodiments, the composition for use in preventing CRF in a feline with hyperthyroidism further comprises from about 0.75 to about 1% potassium. In some such embodiments, the composition comprises from about 0.8 to about 1% potassium. In other such embodiments, the composition comprises from about 0.75 to about 0.9% potassium. Potassium as used herein refers to the potassium atom without reference to its molecular or ionic form. Normally, the majority of ingested potassium is excreted in the urine, with the remainder excreted in feces. The proportion of potassium excreted in feces increases in felines with CRF. Such felines typically also have disorders in potassium homeostasis. Feeding such felines a composition for preventing and/or treating CRF can help maintain optimal potassium balance.

In some embodiments, the composition for use in preventing CRF further comprises two or more of phosphorus, sodium, and potassium in any combination of the amounts discussed above.

In some embodiments, a composition for use in preventing CRF in a hyperthyroid feline as described above further comprises an antithyroid agent (i.e., the composition comprises one or more antithyroid agents). An antithyroid agent is a compound, a derivative thereof (e.g., a salt, solvate, or hydrate of the compound), or a composition comprising such compounds and/or derivatives that is used to treat hyperthyroidism. Suitable antithyroid agents include, for example, thioureylenes (e.g., methimazole, propylthiouracil, and carbimazole), aniline derivatives (e.g., sulfonamides), poryhydric phenols (e.g., resorcinol), and lithium salts. In some embodiments, the antithyroid agent comprises a thioureylene. Thioureylenes are rive- or six-member thiourea derivatives that block production of thyroid hormones. In some such embodiments, the antithyroid agent comprises the thioureylene methimazole. In other such embodiments, the antithyroid agent comprises the thioureylene propylthiouracil. In further such embodiments, the antithyroid agent comprises the thioureylene carbimazole.

In some embodiments, a composition for use according to the present invention comprises a therapeutically- effective amount of an antithyroid agent (*i.e.,* the composition comprises one or more antithyroid agents, and the total amount of the antithyroid agents is a therapeutically-effective amount). A therapeutically-effective or effective amount is an amount that will achieve the .goal of treating the targeted condition. Those skilled in the art either know or can determine by routine experimentation how much of an agent or combination of agents to administer to a feline to treat a specific condition (*e.g.*, hyperthyroidism). For example, one skilled in the art can prepare a composition that, when fed to the feline in a maintenance-sufficient amount, typically will deliver a therapeutically-effective amount of the agent(s) present in the composition. In some cases, the amount of the agent may vary with the stage of treatment For example, higher doses of an antithyroid agent may be used in the initial stage of treatment *(i.e.,* the therapeutically-effective amount of the particular agent may vary with the stage of the disease). One skilled in the art can prepare compositions with varying amounts of an antithyroid agent, and then feed those compositions sequentially to deliver the desired amount of antithyroid agent(s) to the feline.

Treatment can help a hyperthyroid feline with CRF to live a healthier, longer, and more active life. Thus, in another aspect, the invention provides a composition for use in treating CRF in a hyperthyroid feline in need of such treatment (i.e., the composition is useful for treating both CEF and hyperthyroidism). Treating CRF includes ameliorating-, suppressing, and/or delaying CRF. One skilled in the art can diagnose CRF (as well as distinguish it from other diseases, for example, a kidney tumor, diabetes, and hyperthyroidism) by utilizing blood and urine tests as well as, for example, radiography, ultrasound, renal biopsy, and/or palpation. In addition to other symptoms, felines with CRF typically have an elevated serum creatinine. A composition for use in treating CRF is suitable for any hyperthyroid feline with CRF. Examples of felines that can be treated with a composition are discussed above in the context of the compositions for preventing CRF.

The composition for use in treating CRF in a feline with hyperthyroidism comprises from about 28 to about 30% protein. The protein present in the composition comprises at least about 75% vegetable protein. The composition also comprises from about 0.1 to less than about 1 mg/kg iodine and from about 0.1 to about 1 mg/kg iodine.

In some embodiments, the composition use in for treating CRF in a feline with hyperthyroidism comprises from about 28 to about 29% protein, and in other embodiments, the composition comprises from about 29 to about 30% protein. In yet other embodiments, the composition comprises from about 28 or about-28.5 to about 29, about 29.5, or about 30% protein.

In some embodiments, the composition for use in treating CRF in a feline with hyperthyroidism comprises at least about 80% vegetable protein. In other embodiments, the composition comprises at least about 85% vegetable protein. In other embodiments, the protein comprises at least about 90% vegetable protein. In other embodiments, the protein comprises at least about 95% vegetable protein (i.e., from at least about 95 up to about 100% vegetable protein). In some embodiments, the composition comprises from about 28 or about 28.5 to about 29, about 29.5; or about 30% protein wherein the protein comprises at least about 75, at least about 80, at least about 85, at least about 90, or at least about 95% vegetable protein.

In some embodiments, the composition for use in treating CRF in a feline with hyperthyroidism comprises from about 0.1 to less than about 1 mg/kg iodine. In some such embodiments, the composition comprises from about 0.1 to about 0.5 mg/kg iodine. In other such embodiments, the composition comprises from about 0.1 to about 0.3 mg/kg iodine. In yet other such embodiments, the composition comprises from about 0.15 to about 0.25 mg/kg iodine. And in further such embodiments, the composition comprises from about 0.1 to about 0.2 mg/kg iodine.

Additionally, the composition for use in treating CRF in a feline with hyperthyroidism comprises from about 0.1 to about 1 mg/kg selenium. In some such embodiments, the composition comprises from about 0.1 to about 0.8 mg/kg selenium. In other such embodiments, the composition comprises from about 0.15 to about 0.65 mg/kg selenium. In yet other such embodiments, the composition comprises from about 0.4 to about 0.7 mg/kg selenium. In further such embodiments, the composition comprises from about 0.3 to about 0.65 mg/kg selenium.

In some embodiments, the composition for use in treating CRF in a feline with hyperthyroidism comprises from about 0.1 to less than about 1 mg/kg iodine as well as from about 0.1 to less than about 1 mg/kg selenium. In some such embodiments, the composition comprises from about 0.1 to about 0.5 mg/kg iodine and from about 0.1 to about 0.8 mg/kg selenium. In other such embodiments, the composition comprises from about 0.1 to about 0.3 mg/kg iodine and from about 0.15 to about 0.65 mg/kg selenium. In further such embodiments, the composition comprises (1) from about 0.1, about 0.15, or about 0.2 to about 0.25, about 0.3, or about 0.5 mg/kg iodine, and (2) from about 0.1, about 0.15, about 0.3, or about 0.4 to about 0.65, about 0.7, or about 0.8 mg/kg selenium.

One skilled in the art would understand that a composition for use in treating CRF in a feline with hyperthyroidism can comprise any of the combinations of protein (with varying amounts of vegetable protein), iodine, and selenium discussed above.

For example, in some embodiments, the composition for use in treating CRF in a feline with hyperthyroidism comprises from about 28 or about 28.5 to about 29, about 29.5, or about 30% protein wherein the protein comprises at least about 75, at least about 80, at least about 85, at least about 90, or at least about 95% vegetable protein, and (1) from about 0.1, about 0.15, or about 0.2 to about 0.25, about 0.3; or about 0.5 mg/kg iodine, and/or (2) from about 0.1, about 0.15, about 0.3, or about 0.4 to about 0.65, about 0.7, or about 0.8 mg/kg selenium.

In some embodiments, the composition for use in treating CRF in a hyperthyroid feline further comprises from about 0.45 to about 0.6% phosphorus. In some such embodiments, the composition comprises from about 0.45 to about 0.55% phosphorus; and in other such embodiments from about 0.45 to about 0.5% phosphorus.

In some embodiments, the composition for use in treating CRF in a hyperthyroid feline further comprises from about 0.2 to about 0.25% sodium.

In some embodiments, the composition for use in treating CRF in a hyperthyroid feline further comprises from about 0.75 to about 1% potassium. In some such embodiments, the composition comprises from about 0.7 to about 0.9% potassium; and in other such embodiments from about 0.75 to about 0.85% potassium.

In some embodiments, the composition for use in treating CRF in a feline with hyperthyroidism further comprises two or more of phosphorus, sodium, and potassium in any combination of the amounts discussed above.

In some embodiments, a composition for use in treating CRF in a hyperthyroid feline as described above further comprises an antithyroid and/or an anti-CRF agent (*i.e.,* the composition comprises one or more antithyroid agents and/or one or more anti-CRF agents). An anti-CRF agent is a compound, a derivative thereof (*e.g*., a salt, solvate, or hydrate of the compound), or a composition comprising such compounds and/or derivatives that is used to treat CRF. Suitable anti-CRF agents include, for example, agents that reduce blood pressure or protein loss in urine, anabolic steroids, antibiotics, agents that treat anemia, and agents that control vomiting. Suitable antithyroid agents are discussed above in the context of the compositions for use in treating CRF in a feline with hyperthyroidism. In some embodiments, a composition as described herein comprises a therapeutically- effective amount of an anti-CRF agent (*i.e.,* the composition comprises one or more anti-CRF agents, and the total amount of the anti-CRF agents is a therapeutically-effective amount). In some embodiments, a composition for use in treating CRF in a feline with hyperthyroidism comprises a therapeutically-effective amount of an antithyroid agent. In some embodiments, a composition as defined herein comprises a therapeutically-effective amount of an antithyroid agent and a therapeutically-effective amount of an anti-CRP agent.

In some embodiments, the compositions for use in preventing and/or treating CRP comprise a food composition *(i.e.,* the compositions comprise one or more food compositions). In some embodiments, the compositions meet, the AAFCO's minimum nutrient level requirements for reproduction or maintenance. Income embodiments, the food compositions comprise a dry food. In some embodiments, the food compositions comprise a semi-moist food. In some embodiments, the food compositions comprise a moist food. In some embodiments, the food compositions comprise a treat, snack, supplement, or partially or fully edible toy. In some embodiments, the compositions comprise a mixture of one or more foods.

In another aspect, the description provides methods for preparing compositions for preventing and/or treating CRF in a feline with hyperthyroidism. Such composition can be prepared, for example, by mixing various ingredients (including food compositions) that, when combined, yield a composition as described herein. Compositions can also be prepared by the methods discussed in, for example. Small Animal Nutrition, pages 127-146 (2000). As discussed above, plant ingredients suitable for preparing compositions include, for example, potato concentrate, soy concentrate, soybean meal, soy protein isolate, corn gluten meal, rice protein isolate, pea protein concentrate, wheat protein concentrate, and wheat protein isolate. To prepare a low selenium-comprising composition as defined herein, one can use, for example, a selenium-free mineral mix and ingredients that contain small amounts of selenium such as, for example, potato concentrate, soy concentrate, and soy protein isolate. To prepare a low iodine-comprising composition as defined herein, one can, for example, avoid food colorings rich in iodine and can use an, iodine-free mineral mix, non-iodized salt, or ingredients that contain small amounts of iodine such as, for example, potato concentrate, soy concentrate, and soy protein isolate. Iodine and selenium-containing ingredients suitable for preparing a composition of the invention are listed in, for example, table 3 of U.S. Patent Application No. US 2005/0058691 A1. Plant ingredients suitable for preparing a composition include, for example, soybean meal, corn gluten meal, rice protein isolate, pea protein concentrate, wheat protein concentrate, and wheat protein isolate. Eggs can be used for preparing a composition as well. Meat (including fish) ingredients suitable for preparing a composition include, for example, pork liver, beef spleen, beef tongue, pork lung lobes, beef lung, meat protein isolate, deboned turkey, chicken backs, mackerel, oceanfish, and poultry by-product meal. One skilled in the art would understand that in many embodiments of the invention, one would need to utilize mostly plant ingredients to achieve high vegetable protein content as well as low iodine and/or selenium content. As discussed above, commercially available cat foods typically contain higher amounts of iodine and selenium than the amounts of iodine and/or selenium in a composition of the invention. To minimize iodine and/or selenium carryover, before preparing a composition as defined herein, it may be desirable to clean the equipment that will be used. For example, to minimize iodine or selenium carryover from an earlier retort or extrusion run, the equipment can be appropriately flushed before a low iodine and/or low selenium-comprising composition will be made. In some cases, it may also be desirable to discard the initial portion of a batch to obtain a composition with a consistent concentration of iodine and/or selenium throughout the entire batch.

In another aspect, the description provides a method for preventing CRF in a hyperthyroid feline susceptible to develop CRF. In some embodiments, the method comprises feeding the feline a composition selected from the compositions for preventing CRF in a hyperthyroid feline. One skilled in the art would understand that cither a single composition can be fed to the feline or, alternatively, different such compositions can be fed to the feline for varying time intervals.

In some aspects, the method for preventing CRF in a feline with hyperthyroidism comprises feeding the feline a composition comprising (I) from 28 to about 35% protein wherein the protein comprises at least about 75% vegetable protein, and (2) from about 0.1 to less than about 1 mg/kg iodine and/or from about 0.1 to about 1 mg/kg selenium.

In some such aspects, the method for preventing CRF in a hyperthyroid feline comprises feeding the feline a composition comprising from about 29 to about 31% protein wherein the protein comprises at least about 75, at least about 80, at least about 85, at least about 90, or at least about 95% vegetable protein, and (1) from about 0.1, about 0.15, or about 0.2 to about 0.25, about 0.3, or about 0.5 mg/kg iodine, and/or (2) from about 0.1, about 0.15, about 0.3, or about 0.4 to about 0.65, about 0.7, or about 0.8 mg/kg selenium.

In some such aspects, the method for preventing CRF in a hyperthyroid feline comprises feeding the feline a composition comprising from about 28 or about 28.5 to about 29, about 29.5, about 30, about 30.5, or about 31% protein wherein the protein comprises at least about 75, at least about 80, at least about 85, at least about 90, or at least about 95% vegetable protein, and (1) from about 0.1, about 0.15, or about 0.2 to about 0.25, about 0.3, or about 0.5 mg/kg iodine, and/or (2) from about 0.1, about 0.15, about 0.3, or about 0.4 to about 0.65, about 0.7, or about 0.8 mg/kg selenium.

In other such aspects, the method for preventing CRF in a feline with hyperthyroidism comprises feeding the feline a composition comprising from about 31, about 32, or about 33 to about 34 or about 35% protein wherein the protein comprises at least about 75, at least about 80, at least about 85, at least about 90, or at least about 95% vegetable protein, and (1) from about 0.1, about 0.15, or about 0.2 to about 0.25, about 0.3, or about 0.5 mg/kg iodine, and/or (2) from about 0.1, about 0.15, about 0.3, or about 0.4 to about 0.65, about 0.7, or about 0.8 mg/kg selenium.

In other such aspects, the method for preventing CRF in a feline with hyperthyroidism comprises feeding the feline a composition comprising from about 31 to about 32, about 33, about 34, or about 35% protein, and the protein comprises at least about 75, at least about 80, at least about 85, at least about 90, or at least about 95% vegetable protein, and (1) from about 0.1, about 0.15, or about 0.2 to about 0.25, about 0.3, or about 0.5 mg/kg iodine, and/or (2) from about 0.1, about 0.15, about 0.3, or about 0.4 to about 0.65, about 0.7, or about 0.8 mg/kg selenium.

In other such aspects, the method for preventing CRF in a feline with hyperthyroidism comprises feeding the feline a composition comprising from about 28 or about 28.5 to about 29, about 29.5, or about 30% protein, and the protein comprises at least about 75, at least about 80, at least about 85, at least about 90, or at least about 95% vegetable protein, and (1) from about 0.1, about 0.15, or about 0.2 to about 0.25, about 0.3, or about 0.5 mg/kg iodine, and/or (2) from about 0.1, about 0.15, about 0.3, or about 0.4 to about 0J65, about 0.7, or about 0.8 mg/kg selenium.

In some aspects of the method for preventing CRF in a feline with hyperthyroidism, the composition that is fed to the feline further comprises from about 0.45 to about 0.8% phosphorus. In some such, embodiments, the composition that is fed to the feline -comprises from about 0.45 to about 0.6% phosphorus, for example, from about 0.45 to about 0.55% or from about 0.45 to about 0.5% phosphorus. In other such embodiments, the composition fed to the feline comprises from about 0.6 to about 0.8% phosphorus, for example, from about 0.7 to about 0.8% or from about 0.6 to about 0.75% phosphorus.

In some aspects of the method for preventing CRF in a feline with hyperthyroidism, the composition that is fed to the feline further comprises from about 0.2 to about 0.35% sodium. In some such embodiments, the composition fed to the feline comprises from about 0.2 to about 0.25% sodium, and in other such embodiments, the composition fed to the feline comprises from about 0.2 to about 0.25% sodium.

In some aspects of the method for preventing CRF in a feline with hyperthyroidism, the composition that is fed to the feline further comprises from about 0.75 to about 1% potassium. In some such embodiments, the composition fed to the feline comprises from about 0.8 to about 1% potassium, and in other such embodiments, the composition comprises from about 0.75 to about 0.9% potassium.

In some aspects of the method for preventing CRF in a feline with hyperthyroidism, the composition fed to the feline further comprises two or more of phosphorus, sodium, and potassium in any combination of the amounts discussed above.

In another aspect, the present description provides a method for treating CRF in a hyperthyroid feline in need of such treatment. In some embodiments, the method comprises feeding the feline a composition selected from the compositions for treating CRF in a hyperthyroid feline. One skilled in the art would understand that either a single composition can be fed to the feline or, alternatively, different such compositions can be fed to the feline for varying time intervals.

In some aspects, the method for preventing CRF in a feline with hyperthyroidism comprises feeding the feline a composition comprising (1) from 28 to about 35% protein wherein the protein comprises at least about 75% vegetable protein, and (2) from about 0.1 to less than about 1 mg/kg iodine and/or fronuahout-0.1 to about 1 mg/kg selenium.

In some such aspects, the method comprises feeding the feline a composition comprising from about 28 or about 28.5 to about 29, about 29.5, or about 30% protein wherein the protein comprises at least about 75, at least about 80, at least about 85, at least about 90, or at least about 95% vegetable protein, and (1) from about 0.1, about 0.15, or about 0.2 to about 0.25, about 03, or about 0.5 mg/kg iodine, and/or (2) from about 0.1, about 0; 15, about 0.3, or about 0.4 to about 0.65, about 0.7, or about 0.8 mg/kg selenium.

In some aspects of the method for treating CRF in a feline with hyperthyroidism, the composition that is fed to the feline further comprises from about 0.45 to about 0.6% phosphorus. In some such embodiments, the composition fed to the feline comprises from about 0.45 to about 0.55% phosphorus. In other such embodiments, the composition fed to the feline comprises from about 0.45 to about 0.5% phosphorus.

In some aspects of the method for treating CRF in a feline with hyperthyroidism, the composition that is fed to the feline further comprises from about 0.2 to about 0.25% sodium.

In some aspects of the method for treating CRF in a feline with hyperthyroidism, the composition -that is fed to the feline further comprises from about 0.75 to about 1% potassium. In some such embodiments, the composition that is fed to the feline comprises from about 0. to about 0.9% potassium. In other such embodiments, the composition fed to the feline comprises from about 0.75 to about 0.85% potassium.

In some aspects of the method for treating CRF in a feline, the composition fed to the feline further comprises two or more of phosphorus, sodium, and potassium in any combination of the amounts discussed above.

In some aspects, the methods for prevention and treatment of CRF in a feline with hyperthyroidism further comprise administering to the feline an anti-CRF agent. In some embodiments, the methods for prevention and treatment of CRF in a feline with hyperthyroidism further comprise administering to the feline an antithyroid agent. In some aspects, the methods for prevention and treatment of CRF in a feline with hyperthyroidism further comprise administering to the feline an anti-CRF agent and an antithyroid agent. Such agents are administered in conjunction with feeding a composition as defined herein. The term "in conjunction" means that an agent is administered to the feline either together with a composition as defined herein or separately from the composition at the same or different frequency via the same or different administration route and either at about the same time as the composition or periodically. "Administering" means that the agent is introduced in a suitable dosage form into the feline by a suitable administration route, for example, orally, topically, or parenterally. "About at the same time" generally means that an agent is administered when a composition is fed to the feline or within about 72 hours of feeding the composition to the feline. "Periodically" generally means that an agent is administered to a feline following a dosage schedule-suitable for administering the agent while a composition is fed to the feline routinely as appropriate for that feline. Thus, the term "in conjunction" specifically includes situations when an agent is administered to a feline for a prescribed period of time while a composition is fed to the feline for a much longer period of time (*e.g*., for life). If more than one agent is administered, the dosage form and route of administration for each agent .may vary. In addition, one composition may be substituted with another composition while a specific agent is administered to the feline.

Suitable anti-CRF and antithyroid agents are discussed above in the context of the compositions as defined herein. Such agents can be administered, for example, in the form of salts derived from inorganic or organic acids. Depending on the particular compound, a salt of the compound may be advantageous due to one or more of the salt's physical properties, for example, enhanced pharmaceutical stability in differing temperatures and humidities, or a desirable solubility in water or oil. The salt preferably is a pharmaceutically-acceptable salt.

The total daily dose of an agent (administered in single or divided doses) is typically from about 0.001 to about 100 mg/kg, preferably from about 0.001 to about 30 mg/kg, and even more preferably from about 0.01 to about 10 mg/kg body weight. Dosage unit compositions can contain such amounts or submultiples thereof to make up the daily dose. In many instances, the administration of an agent will be repeated a plurality of times. Multiple doses per day typically may be used to increase the total daily dose. Factors affecting the preferred dosage regimen include, for example, the age, weight, and condition of the feline; the severity of the disease; the route of administration; pharmacological considerations (*e.g*., activity, efficacy, and pharmacokinetic and toxicology profiles of the particular agent); whether a drug delivery system is utilized; and whether the agent is administered as part of a drug combination. Thus, the dosage regimen actually employed can vary widely, and can differ from the dosage regimen set forth above.

In another aspect, the description provides a use of a composition *(i.e.,* any composition for use in preventing CRF in a feline discussed above) to prepare a medicament to prevent CRF in a feline with hyperthyroidism.

In another aspect, the description provides a use of a composition *(i.e.,* any composition for use in treating CRF in a feline as discussed above) to prepare a medicament to treat CRF in a feline.

In a further aspect, the description provides an article of manufacture, for example, a kit comprising a composition as discussed above. The kit is suitable for preventing and/or treating CRF in a feline with hyperthyroidism. In some aspects, the kit further comprises an anti-CRF agent *(i.e.,* one or more anti-CRF agents). In some aspects, the kit further comprises an antithyroid agent (i.e., one or more antithyxoid-agents). In some aspects, the kit further comprises an anti-CRF agent and an antithyroid agent In some embodiments, the kit further comprises instructions for, for example, one or more of (a) feeding the composition to a feline with hyperthyroidism, (b) administering an anti-CRF agent and/or an antithyroid agent to the feline in conjunction with feeding the feline the composition, (c) preventing CRF in a feline with hyperthyroidism by feeding the feline the composition, (d) treating CRF in a feline with hyperthyroidism by feeding the feline the composition, (e) preventing CRF in a feline with hyperthyroidism by administering to the feline an anti-CRF agent and/or an antithyroid agent in conjunction with feeding the feline the composition, and (f) treating CRF in a feline with hyperthyroidism by administering to the feline, an anti-CRF agent and/or an antithyroid agent in conjunction with feeding the feline the composition.

In some aspects of the description, the kit comprises two or more ingredients that, when combined together and optionally with additional Ingredients that are or are not a part of the kit, yield a composition of the present invention. If such additional ingredients are to be used, the kit provides instructions about these ingredients. In some aspects, the kit further comprises an anti-CRF agent. In some aspects, the kit further comprises an antithyroid agent. In some embodiments, the kit further comprises an anti-CRF agent and an antithyroid agent. In some embodiments, the kit further comprises instructions for one or more of (a) preparing a composition as described herein for preventing CRF in a hyperthyroid feline by combining the ingredients, (b) preparing a composition for treating CRF in a hyperthyroid feline by combining the ingredients, (c) feeding a feline with hyperthyroidism a composition for preventing CRF, (d) feeding a feline with hyperthyroidism a composition for treating CRF, (e) administering an anti-CRF agent and/or an antithyroid agent to a feline in conjunction with feeding the feline a composition for preventing CRF, (f) administering an anti-CRF agent and/or an antithyroid agent to a feline with hyperthyroidism in conjunction with feeding the feline a composition for treating CRF, (g) preventing CRF in a feline with hyperthyroidism by feeding the feline a composition for preventing CRF, (h) treating CRF in a feline with hyperthyroidism by feeding the feline a composition for treating CRF, (i) preventing CRF in a feline with hyperthyroidism by administering to the feline an anti-CRF agent and/or an antithyroid agent in conjunction with feeding the feline a composition, for preventing CRF, and (j) treating CRF in a feline with hyperthyroidism by administering to the feline an anti-CRF agent and/or an antithyroid agent in conjunction with feeding the feline a composition for treating CRF.

In some aspects, the kit comprises in separate containers in a single package or in separate containers in a virtual package, as appropriate, a composition or two or more ingredients, that, when combined together and optionally with additional ingredients that are or are not a part of the kit, yield a composition as defined herein, and instructions for one or more of (a) preparing a composition of the invention, for preventing CRF in a feline with hyperthyroidism by combining the ingredients, (b) preparing a composition for treating CRF in a feline with hyperthyroidism by combining the ingredients, (c) feeding a feline with hyperthyroidism a composition to prevent CRF, (d) feeding a feline with hyperthyroidism a composition to treat CRF, (e) administering an anti-CRF agent to a feline with hyperthyroidism in conjunction with feeding the feline a composition to prevent CRF, (f) administering an anti-CRF agent and/or an antithyroid agent to a feline with hyperthyroidism in conjunction with feeding the feline a composition to treat CRF, (g) preventing CRF in a feline with hyperthyroidism by feeding the feline a composition for preventing CRF, (h) treating CRF in a feline with hyperthyroidism by feeding the feline a composition for treating CRF, (i) preventing CKF in a feline with hyperthyxoidism by administering to the feline an anti-CRF agent and/or an antithyroid agent in conjunction with feeding the feline a composition for preventing CRF, and (j) treating CRF in a feline with hyperthyroidism by administering to the feline an anti-CRF agent and/or an antithyroid agent in conjunction with feeding the feline a composition for treating CRF.

The term "single package" generally means that the components of a kit are physically associated in or with one or more containers, and considered as a unit of manufacture, distribution, sale, or use. Containers include, for example, bags, boxes, bottles, shrink wrap packages, stapled or otherwise fixed components, and combinations thereof. A single, package can be, for example, containers or individual food compositions physically associated such that they are considered a unit for manufacture, distribution, sale, or use. The term "virtual package" generally means that the components of a kit are associated by directions on one or more physical or virtual kit components instructing the user how to obtain additional components, e.g., in a bag containing one component and directions instructing the user to go to a website, contact a recorded message, view a visual message, or contact a caregiver to obtain instructions on how to use the kit. When the kit comprises a virtual package, the kit is limited to instructions in a virtual environment with one or more physical kit components.

In some aspects, the kit is suitable for preventing chronic renal failure in a feline with hyperthyroidism and comprises in separate containers in a single package or in separate containers in a virtual package, as appropriate, a composition comprising (1) from about 28 to about 3 5% protein wherein the protein comprises at least about 75% vegetable protein, and (2) from about 0.1 to less than about 1 mg/kg iodine and/or from about 0.1 to about 1 mg/kg selenium, or two or more ingredients that, when combined together and, optionally, with additional ingredients that are or are not a part of the kit, yield a composition comprising from about 28 to about 35% protein wherein the protein comprises at least about 75% vegetable protein, and (2) from about 0.1 to less than about 1 mg/kg iodine and/or from about 0.1 to about 1 mg/kg selenium, and one or more of (a) an anti-chronic renal failure agent, (b) antithyroid agent, (c) instructions for preparing the composition, (d) instructions for feeding the composition to a feline with hyperthyroidism, (e) instructions for preventing chronic renal failure in a feline with, hyperthyroidism by feeding the feline the composition, and (f) instructions for preventing chronic renal failure in a feline with hyperthyroidism by administering to the feline an anti-chronic renal failure agent and/or an antithyroid agent in conjunction-with feeding the feline the composition. In some such embodiments, the kit comprises a composition comprising (1) from about 31 to about 35% protein wherein the protein comprises at least about 75% vegetable protein, and (2) from about 0.1 to less than about 1 mg/kg iodine and/or from about 0.1 to about 1 mg/kg selenium, or two or more ingredients that, when combined together and, optionally, with additional ingredients that are or are not-a part of the kit, yield a composition comprising (1) from about 31 to about 35% protein wherein the protein comprises at least about 75% vegetable protein, and (2) from about 0.1 to less than about 1 mg/kg iodine and/or iron, about 0.1 to about 1 mg/kg selenium.

In other aspects, the kit is suitable for treating chronic renal failure in a feline with hyperthyroidism and comprises in separate containers in a single package or in separate containers in a virtual package, as appropriate, a composition comprising (1) from about 28 to about 30% protein wherein the protein comprises at least about 75% vegetable protein, and (2) from about 0.1 to less than about 1 mg/kg iodine and/or from about 0.1 to about 1 mg/kg selenium, or two or more ingredients that, when combined together and, optionally, with additional ingredients that are or are not a part of the kit, yield a composition comprising from (1) from about 28 to about 30% protein wherein the protein comprises at least about 75% vegetable protein, and (2) from about 0.1 to less than about 1 mg/kg iodine and/or from about 0.1 to about 1 mg/kg selenium, and-one or more of (a) an anti-chronic renal failure agent, (b) an antithyroid agent, (c) instructions for preparing the composition, (d) instructions for feeding the composition to a feline with hyperthyroidism, (e) instructions for treating chronic renal failure in a feline with hyperthyroidism by feeding the feline the composition, and (f) instructions for treating chronic renal failure in a feline with hyperthyroidism by administering to the feline an anti-chronic renal failure agent and/or an antithyroid agent in conjunction with feeding the feline the composition.

In a further aspect, the description provides a means for communicating information about or instructions for one or more of (a) feeding a feline with, hyperthyroidism composition to prevent CRF, (b) feeding a feline with hyperthyroidism a composition to treat CRF, (c) administering an anti-CRF agent and/or an antithyroid agent to a feline in conjunction with feeding the feline a composition to prevent CRF, (d) administering an anti-CRF agent and/or an antithyroid agent to a feline with hyperthyroidism in conjunction with feeding the feline a composition to treat CRF, (e) preventing CRF in a feline with hyperthyroidism by feeding the feline a composition for preventing CRF, (f) treating CRF in a feline with hyperthyroidism by feeding the feline a composition for treating CRF, (g) preventing CRF in a feline with hyperthyroidism by administering to the feline an anti-CRF agent and/or antithyroid agent in conjunction with feeding the feline a composition for preventing CRF, (h) treating CRF in a feline with hyperthyroidism by administering to the feline an anti-CRF agent and/or antithyroid agent in conjunction with feeding the feline a composition for treating CRF, (Q using a kit to prevent CRF in a feline with hyperthyroidism, and (j) using a kit to treat CRF in a feline with hyperthyroidism. The communication means comprise, for example, a document, digital storage media, optical storage media, audio presentation, or visual display containing the information or instructions. Preferably, the communication means is a displayed web site or a brochure, product label, package insert, advertisement, or visual display containing such information or instructions. Useful information or instructions include, for example, (1) information and instructions how to use a composition, method or kit as provided herein and (2) contact information for animal caregivers if they have a question about the invention and its uses.

In some aspects, the present description provides a means for communicating information about or instructions for one or more of (a) preventing chronic renal failure in a feline with hyperthyroidism by feeding the feline a composition comprising (1) from about 28 to about 35% protein wherein the protein comprises at least about.75% vegetable protein, and (2) from about 0.1 to less than about 1 mg/kg iodine and/or from about 0.1 to about 1 mg/kg selenium; (b) preventing chronic renal failure in a feline with hyperthyroidism by administering to the feline an anti-chronic renal failure agent and/or an antithyroid agent in conjunction with feeding the feline a composition comprising (1) from about 28 to about 35% protein wherein the protein comprises at least about 75% vegetable protein, and (2) from about 0.1 to less than about 1 mg/kg iodine and/or from- about 0.1 to about 1 mg/kg selenium; and (c) using a kit comprising a composition, comprising (1) from about 28 to about 35% protein wherein the protein comprises at least about 75% vegetable protein, and (2) from about 0.1 to less than about 1 mg/kg iodine and/or from about 0.1 to about 1 mg/kg selenium, or two or more ingredients that when combined together and, optionally, with additional ingredients, yield a composition comprising (1) from about 28 to about 35% protein wherein the protein comprises at least about 75% vegetable protein, and (2) from about 0.1 to less than about 1 mg/kg iodine and/or from about 0.1 to about I mg/kg selenium. The means comprise a document, digital storage media, audio presentation, or visual display containing the information or instructions.

In other aspects, the present description provides a means for communicating information about or instructions for one or more of (a) treating chronic renal failure in a feline with hyperthyroidism by feeding the feline a composition comprising (1) from about 28 to about 30% protein wherein the protein comprises at least about 75% vegetable protein, and (2) from about 0.1 to less than about 1 mg/kg iodine and/or from about 0.1 to about 1 mg/kg selenium; (b) treating chronic renal failure in a feline with hyperthyroidism by administering to the feline and anti-chronic renal failure agent and/or an antithyroid agent in conjunction with feeding the feline a composition comprising (1) from about 28 to about 30% protein wherein the protein comprises at least about 75% vegetable protein, and (2) from about 0.1 to less than about 1 mg/kg iodine and/or from about 0.1 to about 1 mg/kg selenium; and (c) using a kit comprising a composition comprising (1) from about 28 to about 30% protein wherein the protein comprises at least about 75% vegetable protein, and (2) from about 0.1 to less than about 1 mg/kg iodine and/or from about 0.1 to about 1 mg/kg selenium, or two or more ingredients that when combined together and, optionally, with additional ingredients, yield a composition comprising (1) from about 28 to about 30% protein wherein the protein comprises at least about 75% vegetable protein, and (2) from about 0.1 to less than about 1 mg/kg iodine and/or from about 0.1 to about 1 mg/kg selenium. The communication means comprise a document, digital storage media, audio presentation, or visual display containing the information or instructions.

In other aspects, the description provides a use of any of the compositions as defined herein in the manufacture of a food composition for the treatment and/or prevention of chronic renal failure in an animal. Specifically provided is a use of vegetable protein, selenium and iodine in the manufacture of a food composition for the treatment and/or prevention of chronic renal failure in an animal comprising from 25% to 35% protein on a dry matter basis wherein the protein is at least 75% vegetable protein, from 0.1 mg/kg to 1 mg/kg iodine, and from 0.1 to 1 mg/kg selenium on a dry matter basis. Such an animal, e.g., a feline (e.g., a cat), may be suffering from hyperthyroidism.

### Examples

The invention can be further illustrated by the following examples, although it will be understood that the examples are included merely for purposes of illustration and are not intended to limit the scope of the invention unless otherwise specifically indicated.

### Example 1

Example 1 illustrates the effect of the compositions of the intervention on preventing and/or treating CRF concurrently with treating hyperthyroid cats.

Food B1 is formulated as a dry cat food containing 0.6% selenium. Food B1 comprises soybean meal, corn gluten meal, poultry meal, and pork meat protein isolate as the protein ingredients, and is formulated with both iodized and non-iodized salt but no selenium. The average moisture content is 6.6%, the average protein content is 33.7%, and the average iodine content is 0.38% mg/kg (ten samples are taken for iodine analysis, and the iodine content of those samples vary from 0.27 to 0.60 mg/kg).

Food B2 is formulated as a wet cat food containing 0.6% selenium. It comprises soybean meal, pork lungs, chicken, and pork liver as the protein ingredients. No source of iodine is intentionally added to food B2. No selenium salts are added to food B1. The average moisture content for those samples is 78.2%, the average protein content is 33.2%, and the average iodine content is 0.21 mg/kg (ten samples are taken for iodine analysis, and the iodine content of those samples vary from 0.14 to 0.27 mg/kg).

Food B1 and food B2 are mixed in a 1:1 ratio, thus resulting in food B. A group of five cats diagnosed with hyperthyroidism are fed food B for six weeks. Another five cats diagnosed with hyperthyroidism are given 2.5 mg methimazole orally once a day for six weeks while they are fed food B. Eight cats diagnosed with hyperthyroidism are given 2.5 mg methimazole orally once a day for six weeks while they are fed a commercially available cat food. The thyroid hormone profiles and serum chemistries of all cats are measured at zero, two, four, and six weeks. All cats enrolled in the study have elevated total T₄ and/or free T₄, with the majority of the cats exhibiting one or more clinical signs associated with hyperthyroid disease *(e.g*., weight loss, heart murmur/tachycardia, unkempt hair coat, thyroid gland enlargement, increased appetite, vomiting, increased activity, diarrhea, polyuria/polydipsia, aggressiveness, and panting). The results from the study are presented in Tables 1 and 2.

**Table 1**

| Change in Serum Total T₄ Levels | |
|---|---|
| Treatment | Change in Serum Total T₄ Levels (nmol/L) |
| Methimazole | -20.3 |
| Food B | -31.5 |
| Methimazole + Food B | -42.2 |

**Table 2**

| Change in Serum Creatinine Levels | |
|---|---|
| Treatment | Change in Serum Creatinine Levels (mg/dL) |
| Methimazole | +0.46 |
| Food B | -0.08 |
| Methimazole + Food B | -0.03 |

Treatment with 2.5 mg methimazole administered orally once daily for six weeks results in a decrease in serum total T₄ concentration and in an increase in serum creatinine concentration (serum creatinine was a bit higher than the 0.8-1.8 mg/dL normal creatinine concentration range). Although this treatment is effective (the total T₄ concentration was lowered although it did not reach the normal total T₄ concentration range of 10-55 nmol/dL), it may cause or exacerbate kidney disease, and may therefore be unsuitable for treating hyperthyroidism in cats with CRF.

Feeding food B results in a greater decrease in serum total T₄ concentration than treatment with 2.5 mg methimazole alone (although the total T₄ concentration was still a bit higher than the normal total T₄ concentration range). It also results in a decrease in serum creatinine concentration (with creatinine concentration falling within the normal creatinine range). Feeding food B is a more effective treatment for hyperthyroidism than administering 2.5 mg methimazole alone. Feeding food B does not result in the side effects associated with methimazole treatment. Food B is also suitable for felines suffering from or susceptible to developing CRF.

Administering 2.5 mg methimazole while feeding food B results in the greatest decrease in serum total T₄ concentration. That decrease in serum total T₄ concentration is higher than the corresponding decrease for treatment with methimazole only with T₄ falling within the normal total T₄ concentration range, indicating that there is a synergism between the action of food B and the antithyroid agent. Administering methimazole in conjunction with feeding food B also results in a decrease in serum creatinine concentration (with serum creatinine concentration within the normal range), indicating that the combination is suitable for felines with or susceptible to developing CRF.

### Example 2

Examples 2 illustrates the effect of the compositions of the invention on preventing and/or treating CRF concurrently with treating hyperthyroidism in cats.

Fifteen cats diagnosed with hyperthyroidism are fed food B (from Example 1) for twelve weeks. Eleven cats diagnosed with hyperthyroidism are fed a commercially available cat food (i.e., control food) for twelve weeks. The thyroid hormone profiles and serum chemistries of all cats are measured at zero, two, four, six, and twelve weeks. All cats enrolled in the study have elevated total T₄ and/or free T₄, with the majority of the cats exhibiting one or more clinical signs associated with hyperthyroid disease. The results from this study are presented in Tables 3 and 4.

**Table 3**

| Change in Serum Total T₄ Levels | |
|---|---|
| Treatment | Change in Serum Total T₄ Levels (nmol/L) |
| Control Food | +6.5 |
| Food B | -50.4 |

**Table 4**

| Change in Serum Creatinine Levels | |
|---|---|
| Treatment | Change in Serum Creatinine Levels (mg/dL) |
| Control Food | +0.282 |
| Food B | -0.277 |

Feeding control food for twelve weeks results in an increase in both serum total T₄ and serum creatinine concentrations (with the total T₄ concentration above the normal range and the creatinine concentration within the normal range). Feeding food B for twelve weeks results in a decrease in both serum total T₄ and serum creatinine concentrations (with both total T₄ and creatinine concentrations falling within the normal ranges).

Unless otherwise stated, all percentages provided herein are weight percentages on a dry matter basis, e.g., 31% protein means 31% protein on a dry matter basis. Also, the concentrations of ingredients are given, e.g.; 1 mg/kg iodine means 1 mg/kg iodine on a dry matter basis. The term "dry matter basis" means that an ingredient's concentration in a composition is measured after removing the moisture from the composition.

The singular forms "a", "an", and "the" include plural references unless the context clearly indicates otherwise. The term "comprise", "comprises", and "comprising" are to be interpreted inclusively rather than exclusively.

In the specification, there have been disclosed typical preferred embodiments of the invention and, although specific terms are employed, they are used in a generic and descriptive sense only and not for purposes of limitation, the scope of the invention being set forth in the claims. Obviously many modifications and variations of the invention are possible in light of the above teachings. It is therefore to be understood that within the scope of the appended claims the invention may be practiced otherwise than as specifically described.

Unless defined otherwise, all technical and scientific terms and acronyms used herein have the same meanings as commonly understood by one of ordinary skill in the art in the field of the invention. Although any compositions, methods, articles of manufacture, and means for communicating information similar or equivalent to the ones described herein can be used to practice the invention, the preferred compositions, methods, articles of manufacture, and means for communicating information are described herein.

All patents, patent applications, and publications cited herein are incorporated by reference into this patent application to the extent allowed by law. The discussion of those references is intended merely to summarize the assertions made by their authors. No admission is made that any such patents, patent applications, or publications (or a portion thereof) is relevant prior art. Applicants reserve the right to challenge the accuracy and pertinence of the cited patents, patent applications, and publications.

## Claims

1. A composition for use in preventing or treating chronic renal failure in a feline with hyperthyroidism,
wherein if the composition is for use in preventing chronic renal failure, the composition comprises from 28 to 35% protein on a dry matter basis wherein the protein comprises at least 75% vegetable protein; and preferably, wherein the composition comprises from 31 to 35% protein,
wherein if the composition is for use in treating chronic renal failure, the composition comprises from 28 to 30% protein on a dry matter basis, wherein the protein comprises at least 75% vegetable protein,
and wherein the composition for use in preventing or treating chronic renal failure further comprises from 0.1 to less than 1 mg/kg iodine on a dry matter basis and/or from 0.1 to 1 mg/kg selenium on a dry matter basis.

2. The composition for use according to claim 1, wherein the composition is for use in preventing chronic renal failure, and wherein the composition further comprises from 0.6 to 0.8% phosphorus, and/or further comprises from 0.25 to 0.35% sodium, and/or further comprises from 0.75 to 1 % potassium.

3. The composition for use according to claim 1 or claim 2, wherein the composition is for use in preventing chronic renal failure, and wherein the composition comprises from 0.1 to 0.3 mg/kg iodine, and/or from 0.15 to 0.65 mg/kg selenium.

4. The composition for use according to claim 1 or claim 2, wherein the composition is for use in preventing chronic renal failure, and wherein the composition comprises 0.1 to 0.5 mg/kg iodine and from 0.1 to 0.8 mg/kg selenium.

5. The composition for use according to claim 1, wherein the composition is for use in treating chronic renal failure, and wherein the composition
optionally further comprises an antichronic renal failure agent.

6. The composition for use according to claim 5 wherein the composition comprises from 0.1 to 0.3 mg/kg iodine, and/or from 0.15 to 0.65 mg/kg selenium.

7. The composition for use according to claim 5 wherein the composition comprises 0.1 to 0.5 mg/kg iodine and from 0.1 to 0.8 mg/kg selenium.

8. The composition for use according to claims 5 to 7 wherein the composition further comprises from 0.45 to 0.6% phosphorus, and/or further comprises from 0.2 to 0.25% sodium, and/or further comprises from 0.75 to 1 % potassium.

9. A composition for use according to any preceding claim, wherein the composition further comprises an antithyroid agent, and/or wherein the composition is a food composition and/or wherein the feline is a cat.

10. A composition for use according to any of claims 5 - 8 in conjunction with an antithyroid agent as a combined preparation for use in treating chronic renal failure in a feline.

11. The composition for use according to claim 9 or claim 10 wherein the antithyroid agent comprises a thioureylene, preferably selected from the group consisting of methimazole, propylthiouracil, and carbimazole.

12. Use of a combination of vegetable protein, selenium and iodine in the manufacture of a food composition to treat and/or prevent chronic renal failure in a feline having hyperthyroidism, wherein the amount of protein in the composition is from 25% to 35% protein on a dry matter basis, which comprises at least 75% vegetable protein, wherein the amount of selenium in the composition is 0.1 to 1 mg/kg selenium on a dry matter basis, and
wherein the amount of iodine in the composition is 0.1 to 1 mg/kg iodine on a dry matter basis.

## Patentansprüche

1. Zusammensetzung zur Verwendung bei der Vorbeugung oder Behandlung von chronischem Nierenversagen bei einer Katze mit Schilddrüsenüberfunktion,
wobei, wenn die Zusammensetzung zur Verwendung bei der Vorbeugung von chronischem Nierenversagen vorgesehen ist, die Zusammensetzung von 28 % bis 35 % Protein auf einer Trockensubstanzbasis enthält, wobei das Protein mindestens 75 % pflanzliches Protein enthält; und bevorzugt, wobei die Zusammensetzung von 31 % bis 35 % Protein enthält,
wobei, wenn die Zusammensetzung zur Verwendung bei der Behandlung von chronischem Nierenversagen vorgesehen ist, die Zusammensetzung von 28 % bis 30 % Protein auf einer Trockensubstanzbasis enthält, wobei das Protein mindestens 75 % pflanzliches Protein enthält;
und wobei die Zusammensetzung zur Verwendung bei der Vorbeugung oder Behandlung von chronischem Nierenversagen ferner von 0,1 mg/kg bis weniger als 1 mg/kg Iod auf einer Trockensubstanzbasis und/oder von 0,1 mg/kg bis 1 mg/kg Selen auf einer Trockensubstanzbasis enthält.

2. Zusammensetzung zur Verwendung nach Anspruch 1, wobei die Zusammensetzung zur Verwendung bei der Vorbeugung von chronischem Nierenversagen vorgesehen ist, und wobei die Zusammensetzung ferner von 0,6 % bis 0,8 % Phosphor enthält, und/oder ferner von 0,25 % bis 0,35 % Natrium enthält, und/oder ferner von 0,75 % bis 1 % Kalium enthält.

3. Zusammensetzung zur Verwendung nach Anspruch 1 oder Anspruch 2, wobei die Zusammensetzung zur Verwendung bei der Vorbeugung von chronischem Nierenversagen vorgesehen ist, und wobei die Zusammensetzung von 0,1 mg/kg bis 0,3 mg/kg Iod und/oder von 0,15 mg/kg bis 0,65 mg/kg Selen enthält.

4. Zusammensetzung zur Verwendung nach Anspruch 1 oder Anspruch 2, wobei die Zusammensetzung zur Verwendung bei der Vorbeugung von chronischem Nierenversagen vorgesehen ist, und wobei die Zusammensetzung 0,1 mg/kg bis 0,5 mg/kg Iod und von 0,1 mg/kg bis 0,8 mg/kg Selen enthält.

5. Zusammensetzung zur Verwendung nach Anspruch 1, wobei die Zusammensetzung zur Verwendung bei der Behandlung von chronischem Nierenversagen vorgesehen ist, und wobei die Zusammensetzung
gegebenenfalls ferner ein Mittel gegen chronisches Nierenversagen enthält.

6. Zusammensetzung zur Verwendung nach Anspruch 5, wobei die Zusammensetzung von 0,1 mg/kg bis 0,3 mg/kg Iod und/oder von 0,15 mg/kg bis 0,65 mg/kg Selen enthält.

7. Zusammensetzung zur Verwendung nach Anspruch 5, wobei die Zusammensetzung 0,1 mg/kg bis 0,5 mg/kg Iod und von 0,1 mg/kg bis 0,8 mg/kg Selen enthält.

8. Zusammensetzung zur Verwendung nach den Ansprüchen 5 bis 7, wobei die Zusammensetzung ferner von 0,45 % bis 0,6 % Phosphor enthält, und/oder ferner von 0,2 % bis 0,25 % Natrium enthält, und/oder ferner von 0,75 % bis 1 % Kalium enthält.

9. Zusammensetzung zur Verwendung nach einem der vorangehenden Ansprüche, wobei die Zusammensetzung ferner ein antithyreoidales Mittel enthält, und/oder wobei die Zusammensetzung eine Nahrungsmittelzusammensetzung ist, und/oder wobei das feline Tier eine Katze ist.

10. Zusammensetzung zur Verwendung nach einem der Ansprüche 5 bis 8 zusammen mit einem antithyreoidalen Mittel als ein Kombinationspräparat zur Verwendung bei der Behandlung von chronischem Nierenversagen bei einer Katze.

11. Zusammensetzung zur Verwendung nach Anspruch 9 oder Anspruch 10, wobei das antithyreoidale Mittel ein Thioureylen enthält, bevorzugt ausgewählt aus der Gruppe, die aus Methimazol, Propylthiouracil und Carbimazol besteht.

12. Verwendung einer Kombination von pflanzlichem Protein, Selen und Iod bei der Herstellung einer Nahrungsmittelzusammensetzung zur Behandlung und/oder Vorbeugung von chronischem Nierenversagen bei einer Katze mit Schilddrüsenüberfunktion, wobei die Proteinmenge in der Zusammensetzung von 25 % bis 35 % Protein auf einer Trockensubstanzbasis beträgt, das mindestens 75 % pflanzliches Protein enthält, wobei die Selenmenge in der Zusammensetzung 0,1 mg/kg bis 1 mg/kg Selen auf einer Trockensubstanzbasis beträgt, und
wobei die Iodmenge in der Zusammensetzung 0,1 mg/kg bis 1 mg/kg Iod auf einer Trockensubstanzbasis beträgt.

## Revendications

1. Composition pour une utilisation dans la prévention ou le traitement de l'insuffisance rénale chronique chez un félin souffrant d'hyperthyroïdie,
où, si la composition est pour une utilisation dans la prévention de l'insuffisance rénale chronique, elle comprend une teneur en protéines rapportée à la matière sèche qui va de 28 à 35 %, les protéines consistant en au moins 75 % d'une protéine végétale et la composition comprenant préférablement de 31 à 35 % de protéines,
où, si la composition est pour une utilisation dans le traitement de l'insuffisance rénale chronique, elle comprend une teneur en protéines rapportée à la matière sèche qui va de 28 à 30 %, les protéines consistant en au moins 75 % d'une protéine végétale,
et où la composition pour une utilisation dans la prévention ou le traitement de l'insuffisance rénale chronique comprend en outre une teneur en iode rapportée à la matière sèche qui va de 0,1 à moins de 1 mg/kg d'iode et/ou une teneur en sélénium rapportée à la matière sèche qui va de 0,1 à 1 mg/kg.

2. Composition pour une utilisation selon la revendication 1, où la composition est pour une utilisation dans la prévention de l'insuffisance rénale chronique et où la composition comprend en outre de 0,6 à 0,8 % de phosphore et/ou comprend en outre de 0,25 à 0,35 % de sodium et/ou comprend en outre de 0,75 à 1 % de potassium.

3. Composition pour une utilisation selon la revendication 1 ou la revendication 2, où la composition est pour une utilisation dans la prévention de l'insuffisance rénale chronique et où la composition comprend de 0,1 à 0,3 mg/kg d'iode et/ou de 0,15 à 0,65 mg/kg de sélénium.

4. Composition pour une utilisation selon la revendication 1 ou la revendication 2, où la composition est pour une utilisation dans la prévention de l'insuffisance rénale chronique et où la composition comprend de 0,1 à 0,5 mg/kg d'iode et de 0,1 à 0,8 mg/kg de sélénium.

5. Composition pour une utilisation selon la revendication 1, où la composition est pour une utilisation dans le traitement de l'insuffisance rénale chronique et où la composition comprend également en option un agent anti-insuffisance rénale chronique.

6. Composition pour une utilisation selon la revendication 5, où la composition comprend de 0,1 à 0,3 mg/kg d'iode et/ou de 0,15 à 0,65 mg/kg de sélénium.

7. Composition pour une utilisation selon la revendication 5, où la composition comprend de 0,1 à 0,5 mg/kg d'iode et de 0,1 à 0,8 mg/kg de sélénium.

8. Composition pour une utilisation selon les revendications 5 à 7, où la composition comprend en outre de 0,45 à 0,6 % de phosphore et/ou comprend en outre de 0,2 à 0,25 % de sodium et/ou comprend en outre de 0,75 à 1 % de potassium.

9. Composition pour une utilisation selon l'une quelconque des revendications précédentes, où la composition comporte en outre un agent antithyroïdien et/ou où la composition est une composition alimentaire et/ou où le félin est un chat.

10. Composition pour une utilisation selon l'une quelconque des revendications 5 - 8 en conjonction avec un agent antithyroïdien sous forme d'une préparation combinée pour une utilisation dans le traitement de l'insuffisance rénale chronique chez un félin.

11. Composition pour une utilisation selon la revendication 9 ou la revendication 10, où l'agent antithyroïdien comprend un thio-ureylène préférablement sélectionné dans le groupe consistant en le méthimazole, le propylthiouracile et le carbimazole.

12. Utilisation d'une combinaison d'une protéine végétale, de sélénium et d'iode dans la fabrication d'une composition alimentaire pour le traitement et/ou la prévention de l'insuffisance rénale chronique chez un félin souffrant d'hyperthyroïdie, où la teneur en protéines de la composition rapportée à la matière sèche va de 25 % à 35 %, dont 75 % au moins consistent en une protéine végétale, où la teneur en sélénium de la composition rapportée à la matière sèche va de 0,1 à 1 mg/kg et où la teneur en iode de la composition rapportée à la matière sèche va de 0,1 à 1 mg/kg.
